(19) **Europäisches Patentamt / European Patent Office / Office européen des brevets**

(11) **EP 4 656 194 A1**

(12) **EUROPEAN PATENT APPLICATION**
published in accordance with Art. 153(4) EPC

(43) Date of publication:
**03.12.2025 Bulletin 2025/49**

(21) Application number: **24747425.7**

(22) Date of filing: **23.01.2024**

(51) International Patent Classification (IPC):
*A61K 31/519* (2006.01)     *A61K 31/53* (2006.01)
*A61K 31/4985* (2006.01)     *A61K 31/522* (2006.01)
*A61K 31/506* (2006.01)     *A61P 25/00* (2006.01)

(52) Cooperative Patent Classification (CPC):
**A61K 31/4985; A61K 31/506; A61K 31/519;**
**A61K 31/522; A61K 31/53; A61P 25/00**

(86) International application number:
**PCT/KR2024/001065**

(87) International publication number:
**WO 2024/158189 (02.08.2024 Gazette 2024/31)**

(84) Designated Contracting States:
**AL AT BE BG CH CY CZ DE DK EE ES FI FR GB**
**GR HR HU IE IS IT LI LT LU LV MC ME MK MT NL**
**NO PL PT RO RS SE SI SK SM TR**
Designated Extension States:
**BA**
Designated Validation States:
**GE KH MA MD TN**

(30) Priority: **25.01.2023 KR 20230009295**

(71) Applicant: **Aribio Co., Ltd**
**Seongnam-si, Gyeonggi-do 13535 (KR)**

(72) Inventors:
• **CHOUNG, Jaijun**
  **Seongnam-si, Gyeonggi-do 13595 (KR)**
• **SONG, Dongkeun**
  **Chuncheon-si, Gangwon-do 24214 (KR)**
• **NAM, Jusuk**
  **Chuncheon-si, Gangwon-do 24285 (KR)**
• **JUNG, Junsub**
  **Chuncheon-si, Gangwon-do 24230 (KR)**

(74) Representative: **Vossius & Partner**
**Patentanwälte Rechtsanwälte mbB**
**Siebertstrasse 3**
**81675 München (DE)**

(54) **COMPOSITION FOR PREVENTING OR TREATING POST-TRAUMATIC STRESS DISORDER, COMPRISING PHOSPHODIESTERASE TYPE 5 INHIBITOR AS ACTIVE INGREDIENT**

(57)     The present invention relates to a composition for preventing or treating post-traumatic stress disorder, comprising a phosphodiesterase type 5 (PDE 5) inhibitor as an active ingredient, and, particularly, to: a pharmaceutical composition or a health functional food composition for preventing, improving or treating post-traumatic stress disorder, comprising a PDE 5 inhibitor or a pharmaceutically acceptable salt thereof as an active ingredient; and a method for preventing or treating post-traumatic stress disorder, comprising a step of administering the pharmaceutical composition to an individual.

[FIG. 1]

EP 4 656 194 A1

## Description

### Cross-Reference to Related Applications

This application is a national stage entry under 35 U.S.C. §371 of International Application No. PCT/KR2024/001065 filed on January 23, 2024, which claims priority to Korean Patent Application No. 10-2023-0009295 filed on January 25, 2023, the entire contents of each of which are incorporated herein by reference.

### Technical Field

The present invention relates to a composition for preventing or treating post-traumatic stress disorder, comprising a phosphodiesterase type 5 (PDE 5) inhibitor as an active ingredient. More particularly, the present invention relates to a pharmaceutical composition or health functional food composition for preventing, improving or treating post-traumatic stress disorder, comprising a PDE 5 inhibitor or a pharmaceutically acceptable salt thereof as an active ingredient, and a method for preventing or treating post-traumatic stress disorder, comprising a step of administering the pharmaceutical composition to a subject.

### Background

Post-traumatic stress disorder (PTSD) is a mental disorder that was previously classified as an anxiety disorder but has since been reclassified as a trauma- and stressor-related disorder. PTSD may be triggered by exposure to life-threatening or highly traumatic events, including, but not limited to, natural disasters, fires, wars, physical assault, torture, sexual assault, hostage situations, abuse, or other extreme incidents beyond the range of normal human experience. The onset of PTSD symptoms varies among individuals and may occur immediately after the traumatic event or be delayed by days, weeks, months, or even years.

Symptoms of PTSD include hypervigilance (also referred to as hyperalertness) and hyperarousal, re-experience or intrusion of the traumatic event, avoidance or emotional numbness. In addition, patients may exhibit physical symptoms such as headaches, indigestion, vomiting, abdominal pain, tremors, and allergies due to hormonal changes. A wide range of psychological and behavioral symptoms may also occur, including cognitive impairment, memory loss, separation anxiety, school avoidance, and social withdrawal or fear of strangers. In some cases, individuals may develop substance abuse or addiction due to increased dependence on alcohol or drugs, as well as autonomic nervous system disorders. Severe cases may present with hallucinations, dissociative disorders, panic attacks, and other psychiatric complications.

Current treatments for PTSD include cognitive therapy, behavioral therapy, hypnotherapy, group therapy, pharmacotherapy, and nerve block therapy. Among these, pharmacotherapy typically involves the administration of antidepressants, particularly selective serotonin reuptake inhibitors (SSRIs). A representative example of an SSRI is fluoxetine (marketed under the trade name Prozac). It has been reported in various publications that fluoxetine is effective in treating PTSD (see, for example, Journal of Traumatic Stress, 1991, 4(3):419-423; Neurosciences (Riyadh), 2011, 16(3):257-262). Despite the development of various new pharmacological agents for PTSD, fluoxetine remains one of the most widely prescribed drugs in many countries. However, SSRIs such as fluoxetine are associated with adverse effects that limit their long-term use and therapeutic efficacy. These side effects include cognitive impairment, weight gain, sexual dysfunction, sedation, drug dependence, and withdrawal symptoms. Accordingly, there is an ongoing need for the development of safer and more effective drugs suitable for long-term treatment of PTSD.

Meanwhile, phosphodiesterase (PDE) is an enzyme that catalyzes the hydrolysis of cyclic adenosine monophosphate (cAMP) and/or cyclic guanosine monophosphate (cGMP) into 5'-AMP and 5'-GMP, respectively, thereby playing a critical role in the cellular regulation of intracellular cAMP or cGMP levels. To date, a total of 11 PDE isoforms have been identified (see Nature, 2002, pp. 674-682). Among these, PDE 5 is an enzyme that degrades cGMP to 5'-GMP. It has been reported that inhibition of PDE 5 maintains intracellular cGMP levels, thereby promoting and sustaining penile erection (see Boolel, M. et al., British Journal of Urology, 1996, 78:257-261). Accordingly, PDE 5 inhibitors are widely used in the treatment of erectile dysfunction.

A variety of compounds are known as PDE 5 inhibitors. For example, sildenafil (marketed as Viagra™, disclosed in WO 94/28902) was the first PDE 5 inhibitor approved by the U.S. Food and Drug Administration (FDA) for the treatment of male erectile dysfunction. Subsequently, tadalafil (Cialis™, WO 95/19978), vardenafil (Levitra™, Bioorganic & Medicinal Chemistry Letters), and mirodenafil (Mvix™, KR 0358083) were also developed and approved. These agents have demonstrated excellent therapeutic efficacy, improving erectile function in approximately 70% of patients. In addition to their use in the treatment of erectile dysfunction, PDE 5 inhibitors have been reported to be effective for various other medical indications, including portal hypertension, hepatorenal syndrome, and hepatopulmonary syndrome (see Korean Patent Publication No. 10-2012-0024807). Furthermore, their effects on improving reproductive function in mammals have also been disclosed (see Korean Patent Publication No. 10-2002-0031062). However, studies investigating the use of PDE5 inhibitors for the treatment or amelioration of psychiatric disorders, including post-traumatic stress disorder (PTSD), have been extremely limited or insufficient.

## Summary

### Technical Problem

The present inventors have conducted extensive research to develop a pharmaceutical agent for the prevention or treatment of post-traumatic stress disorder (PTSD). As a result, the present invention was completed upon confirmation that phosphodiesterase type 5 (PDE 5) inhibitors, including mirodenafil, sildenafil, and tadalafil, can selectively inhibit contextual fear memory in a trauma- or stress-exposed mouse model (as demonstrated by the Passive Avoidance Test), while preserving novel object recognition ability (as demonstrated by the Novel Object Recognition Test) and spatial memory function (as demonstrated by the Y-maze Test).

The present invention aims to provide a pharmaceutical composition for preventing or treating post-traumatic stress disorder, which comprises a phosphodiesterase type 5 inhibitor or a pharmaceutically acceptable salt thereof as an active ingredient.

The present invention also aims to provide a method for preventing or treating post-traumatic stress disorder, which comprises a step of administering a pharmaceutically effective amount of a composition comprising a phosphodiesterase type 5 inhibitor or a pharmaceutically acceptable salt thereof to a subject.

The present invention also aims to provide a health functional food for preventing or improving post-traumatic stress disorder, which comprises a phosphodiesterase type 5 inhibitor or a pharmaceutically acceptable salt thereof as an active ingredient.

However, the scope of the present invention is not limited to the aforementioned embodiments, and other objectives apparent to those skilled in the art from the following description are also encompassed herein.

### Technical Solution

The present invention provides a pharmaceutical composition or health functional food for the prevention, improvement, or treatment of post-traumatic stress disorder (PTSD), comprising a phosphodiesterase type 5 (PDE5) inhibitor or a pharmaceutically acceptable salt thereof as an active ingredient, and a method for preventing or treating PTSD, comprising administering the pharmaceutical composition to a subject.

According to a first embodiment,
a pharmaceutical composition for preventing or treating PTSD comprising a PDE5 inhibitor or a pharmaceutically acceptable salt thereof as an active ingredient is disclosed.

In the present invention, the PDE5 inhibitor may be selected from the group consisting of mirodenafil, sildenafil, vardenafil, tadalafil, udenafil, dasantafil, avanafil, and pharmaceutically acceptable salts thereof.

In the present invention, the PDE5 inhibitor may be administered via oral administration, intravenous injection, subcutaneous injection, intramuscular injection, intraperitoneal injection, intradermal administration, topical administration, intranasal administration, intravaginal administration, pulmonary administration, rectal administration, or a combination thereof.

In the present invention, a dosage of the PDE5 inhibitor may range from 0.01 mg/kg to 10 mg/kg.

In the present invention, the pharmaceutical composition may further comprise a second agent for the prevention or treatment of PTSD, such as an antidepressant, an anxiolytic, an alpha-1 blocker, or a combination thereof.

In the present invention, the second agent may include sertraline, paroxetine, fluoxetine, citalopram, escitalopram, fluvoxamine, venlafaxine, duloxetine, desvenlafaxine, amitriptyline, nortriptyline, phenelzine, tranylcypromine, mirtazapine, trazodone, bupropion, diazepam, lorazepam, alprazolam, clonazepam, etizolam, metizolam, buspirone, prazosin, doxazosin, or a combination thereof.

In the present invention, the pharmaceutical composition may also be administered in combination with additional therapies such as Eye Movement Desensitization and Reprocessing (EMDR), Cognitive Behavioral Therapy (CBT), exposure therapy, group therapy, or a combination thereof.

According to a second embodiment,
a method for preventing or treating PTSD comprising administering to a subject a pharmaceutical composition comprising a PDE5 inhibitor or a pharmaceutically acceptable salt thereof as an active ingredient is disclosed.

According to a third embodiment,
a health functional food for preventing or improving PTSD comprising a PDE5 inhibitor or a pharmaceutically acceptable salt thereof as an active ingredient is disclosed.

In the present invention, the PDE5 inhibitor may be selected from the group consisting of mirodenafil, sildenafil, vardenafil, tadalafil, udenafil, dasantafil, avanafil, and pharmaceutically acceptable salts thereof.

Advantageous Effects

The PDE5 inhibitor according to the present invention can selectively block context-specific fear memories while preserving novel object recognition and spatial cognitive abilities.

Accordingly, the PDE5 inhibitor of the present invention is expected to exhibit excellent therapeutic effects against PTSD, and may be utilized as an active ingredient in pharmaceutical compositions or health functional foods for the treatment or improvement of PTSD.

## Brief Description of the Drawings

Figure 1 illustrates the effect of administration of a phosphodiesterase type 5 inhibitor on the results of the passive avoidance test in mice.
Figure 2 illustrates the effect of administration of a phosphodiesterase type 5 inhibitor on the results of the novel object recognition test in mice.
Figure 3 illustrates the effect of administration of a phosphodiesterase type 5 inhibitor on the results of the Y-maze test in mice.

## Detailed Description

Hereinafter, a composition for preventing or treating post-traumatic stress disorder (PTSD), comprising a phosphodiesterase type 5 (PDE 5) inhibitor as an active ingredient according to specific embodiments of the present disclosure, will be described in detail. However, the following embodiments are provided for illustrative purposes only, and the scope of the present disclosure is not intended to be limited thereby. It will be understood by those skilled in the art that various modifications may be made without departing from the scope of the present disclosure. Furthermore, the terms "including" or "containing" as used herein are intended to mean the inclusion of a particular component or element without limitation unless otherwise stated, and are not to be construed as excluding the presence or addition of other components.

The term "phosphodiesterase type 5 inhibitor" or "PDE 5 inhibitor" as used herein refers to a substance that selectively or non-selectively inhibits or reduces the catalytic activity of PDE 5. The PDE 5 inhibitor may include compounds, peptides, small molecules, antibodies or fragments thereof, and natural extracts. In one exemplary embodiment, the PDE 5 inhibitor is a compound.

The term "pharmaceutically acceptable salt" as used herein refers to a salt suitable for pharmaceutical use among salts, which are substances formed by electrostatic interactions between cations and anions. Such salts may include, but are not limited to, metal salts, salts with organic bases, salts with inorganic acids, salts with organic acids, and salts with basic or acidic amino acids. For example, metal salts may include alkali metal salts (e.g., sodium salts, potassium salts), alkaline earth metal salts (e.g., calcium salts, magnesium salts, barium salts), aluminum salts, and the like. Salts with organic bases may include salts formed with triethylamine, pyridine, picoline, 2,6-lutidine, ethanolamine, diethanolamine, triethanolamine, cyclohexylamine, dicyclohexylamine, N,N-dibenzylethylenediamine, and the like. Salts with inorganic acids may include salts formed with hydrochloric acid, hydrobromic acid, nitric acid, sulfuric acid, phosphoric acid, and the like. Salts with organic acids may include salts formed with formic acid, acetic acid, trifluoroacetic acid, phthalic acid, fumaric acid, oxalic acid, tartaric acid, maleic acid, citric acid, succinic acid, methanesulfonic acid, benzenesulfonic acid, p-toluenesulfonic acid, and the like. Salts with basic amino acids may include salts formed with arginine, lysine, ornithine, and the like; and salts with acidic amino acids may include salts formed with aspartic acid, glutamic acid, and the like.

The term "active ingredient" as used herein refers to a component that exhibits the desired pharmacological or therapeutic activity on its own, or a component that can exert such activity when administered in combination with a pharmaceutically acceptable carrier that is itself pharmacologically inactive.

The term "post-traumatic stress disorder (PTSD)" as used herein refers to a mental disorder in which abnormal psychosomatic symptoms persist following exposure to a traumatic or shocking event. PTSD is diagnosed when the diagnostic criteria set forth in the Diagnostic and Statistical Manual of Mental Disorders, Fourth Edition (DSM-IV) of the American Psychiatric Association are satisfied. The onset of symptoms may vary between individuals and may occur immediately after the traumatic event or may be delayed by days, weeks, months, or even years. Representative symptoms of PTSD include dissociative phenomena, panic attacks, perceptual disturbances such as auditory hallucinations, aggressive behavior, impulse control disorders, depression, substance abuse, and cognitive impairments including reduced concentration and memory.

The term "prevention" as used herein refers to any action that inhibits or delays the onset of post-traumatic stress disorder (PTSD) by administering the phosphodiesterase type 5 (PDE 5) inhibitor according to the present invention.

The term "treatment" as used herein refers to any action that alleviates, ameliorates, or beneficially modifies one or more symptoms of PTSD in a subject who is suspected of having or is diagnosed with PTSD by administering the PDE 5 inhibitor.

The term "improvement" as used herein refers to any action that at least partially reduces a parameter associated with PTSD, including but not limited to anxiety, hypervigilance, sleep disturbances, or cognitive dysfunction, by administering the PDE 5 inhibitor.

1. Composition for Preventing or Treating Post-Traumatic Stress Disorder

The present invention provides a composition for preventing or treating post-traumatic stress disorder (PTSD), comprising a phosphodiesterase type 5 (PDE5) inhibitor or a pharmaceutically acceptable salt thereof as an active ingredient.

In the composition according to the present invention, the PDE5 inhibitor may be selected from mirodenafil, sildenafil, vardenafil, tadalafil, udenafil, dasantafil, avanafil, or pharmaceutically acceptable salts thereof. In one exemplary embodiment, the PDE5 inhibitor may be mirodenafil, sildenafil, or tadalafil, or a pharmaceutically acceptable salt thereof.

The composition for preventing or treating PTSD according to the present invention may be provided in the form of a pharmaceutical composition, a food composition, or a feed composition.

(1) Pharmaceutical Composition

According to one exemplary embodiment, the present invention provides a pharmaceutical composition for preventing or treating post-traumatic stress disorder, comprising a PDE5 inhibitor or a pharmaceutically acceptable salt thereof as an active ingredient.

In the pharmaceutical composition of the present invention, the pharmaceutical composition may be administered orally or parenterally. The parenteral administration may include, but is not limited to, intravenous injection, subcutaneous injection, intramuscular injection, intraperitoneal injection, endothelial administration, topical administration, intranasal administration, intravaginal administration, intrapulmonary administration, or rectal administration. In the case of oral administration, the pharmaceutical composition may be formulated as a plain (uncoated) tablet, or may be formulated such that the active ingredient is coated or protected against degradation in the stomach (e.g., enteric-coated formulation). Furthermore, the pharmaceutical composition may be administered using any device capable of delivering the active ingredient to a target cell, such as drug delivery systems known in the art. The route of administration may be selected based on various factors, including but not limited to, the age and general condition of the subject, the nature and severity of the condition to be treated, and the specific characteristics of the active ingredient.

In the pharmaceutical composition of the present invention, a suitable dosage of the pharmaceutical composition may vary depending on factors such as the formulation method, route of administration, age, body weight, sex of the subject, pathological condition, diet, administration time, excretion rate, and individual sensitivity to the active ingredient. A person of ordinary skill in the art can readily determine and prescribe an effective dosage for the desired prevention or treatment. For example, the pharmaceutical composition may be administered in a single dose or in multiple divided doses, such as one to four times per day. The dosage may be in the range of 0.01 mg/kg to 10 mg/kg, preferably 0.02 mg/kg to 9 mg/kg, and more preferably 0.03 mg/kg to 8 mg/kg, based on the body weight of an adult.

In the pharmaceutical composition according to the present invention, the composition may further comprise a second active ingredient for preventing or treating post-traumatic stress disorder. The second active ingredient may include an antidepressant, an anxiolytic, or another alpha-1 adrenergic blocker. For example, the antidepressant may include, but is not limited to, a selective serotonin reuptake inhibitor (SSRI), such as sertraline, paroxetine, fluoxetine, citalopram, escitalopram, or fluvoxamine; a serotonin-norepinephrine reuptake inhibitor (SNRI), such as venlafaxine, duloxetine, or desvenlafaxine; a tricyclic antidepressant (TCA), such as amitriptyline or nortriptyline; a monoamine oxidase inhibitor (MAOI), such as phenelzine or tranylcypromine; or an atypical antidepressant, such as mirtazapine, trazodone, or bupropion. The anxiolytic may include, but is not limited to, benzodiazepines such as diazepam, lorazepam, alprazolam, or clonazepam; thienodiazepines such as etizolam or metizolam; or non-benzodiazepine anxiolytics such as buspirone. The alpha-1 adrenergic blocker may include, but is not limited to, prazosin or doxazosin.

In the pharmaceutical composition according to the present invention, the pharmaceutical composition may be used in combination with an additional therapy. For example, the additional therapy may include, but is not limited to, Eye Movement Desensitization and Reprocessing (EMDR), cognitive behavioral therapy (CBT), exposure therapy, group therapy, or any combination thereof.

In the pharmaceutical composition according to the present invention, the pharmaceutical composition may be formulated using a pharmaceutically acceptable carrier and/or excipient according to a method that a person having ordinary skill in the art could easily carry out, thereby to be prepared in a unit dose form or to be contained in a multi-dose container. The above formulation may be a solution in oil or an aqueous medium, a suspension or an emulsion (emulsified solution), an extract, a powder, granules, a tablet, or a capsule, and may further include a dispersing or stabilizing agent. In addition, the pharmaceutical composition may be administered in the form of suppositories, sprays, ointments, creams, gels, inhalants or skin patches. The pharmaceutical composition may also be prepared for mammalian administration, more preferably for human administration.

In the pharmaceutical composition according to the present invention, pharmaceutically acceptable carriers may be in solid or liquid form, and may include one or more selected from the group consisting of fillers, antioxidants, buffers, bacteriostats, dispersing agents, adsorbents, surfactants, binders, preservatives, disintegrants, sweeteners, flavoring agents, glidants, release-controlling agents, wetting agents, stabilizers, suspending agents, and lubricants. In addition, the pharmaceutically acceptable carriers may be selected from saline, sterile water, Ringer's solution, buffered saline, dextrose solution, maltodextrin solution, glycerol, ethanol, and mixtures thereof.

In one embodiment, suitable fillers include, but are not limited to, sugar (e.g., dextrose, sucrose, maltose and lactose), starch (e.g., corn starch), sugar alcohol (e.g., mannitol, sorbitol, maltitol, erythritol and xylitol), starch hydrolysate (e.g., dextrin and maltodextrin), cellulose or cellulose derivatives (e.g., microcrystalline cellulose) or mixtures thereof.

In one embodiment, suitable binders include, but are not limited to, povidone, copovidone, methylcellulose, hydroxypropylmethylcellulose, hydroxypropylcellulose, hydroxyethylcellulose, gelatin, gum, sucrose, starch or mixtures thereof.

In one embodiment, suitable preservatives include, but are not limited to, benzoic acid, sodium benzoate, benzyl alcohol, butylated hydroxyanisole, butylated hydroxytoluene, chlorbutol, gallate, hydroxybenzoate, EDTA or mixtures thereof.

In one embodiment, suitable disintegrants include, but are not limited to, sodium starch glycolate, cross-linked polyvinylpyrrolidone, cross-linked carboxymethylcellulose, starch, microcrystalline cellulose or mixtures thereof.

In one embodiment, suitable sweeteners include, but are not limited to, sucralose, saccharin, sodium saccharin, potassium saccharin, calcium saccharin, acesulfame potassium or sodium cyclamate, mannitol, fructose, sucrose, maltose or mixtures thereof.

In one embodiment, suitable glidants include, but are not limited to, silica, colloidal silicon dioxide, talc and the like.

In one embodiment, suitable lubricants include, but are not limited to, long chain fatty acids and salts thereof, such as magnesium stearate and stearic acid, talc, glyceride wax or mixtures thereof.

(2) food composition

According to one exemplary embodiment, the present invention provides a food composition for preventing or improving post-traumatic stress disorder, comprising a PDE5 inhibitor or a pharmaceutically acceptable salt thereof as an active ingredient.

In the food composition according to the present invention, the food includes, but is not limited to, all foods in a conventional sense, such as meat, sausages, bread, chocolates, candies, snacks, confectionery, pizza, ramen, other noodles, gums, dairy products including ice cream, various soups, beverages, tea, drinks, alcoholic beverages, vitamin complexes, and health functional foods, as long as it can include the PDE5 inhibitor of the present invention. The term "health functional food", as used herein, refers to food manufactured and processed using raw materials or ingredients having functionality useful for the human body according to the Health Functional Food Act No. 6727, and the term "functionality" refers to obtaining useful effects for health purposes, such as regulating nutrients or having physiological effects for the structure and function of the human body. Meanwhile, the 'health food' means a food having an effect of active health maintenance or promotion compared to general food, and the "health supplement food" means a food for the purpose of supplementing health. In some cases, the terms health functional food, health food, and health supplement food may be used interchangeably.

In the food composition according to the present invention, the PDE5 inhibitor may be added as it is or used together with other foods or food ingredients, and may be appropriately used according to conventional methods in the art.

In the food composition according to the present invention, the food composition may be prepared by methods commonly used in the field, and in the course of such preparation, raw materials and components commonly added in the art may be included. Specifically, the food composition may further comprise a physiologically acceptable carrier, wherein the type of the carrier is not particularly limited, and any carrier commonly used in the relevant technical field may be employed. In addition, the food composition may include food additives such as preservatives, disinfectants, antioxidants, coloring agents, color developers, bleaches, seasonings, sweeteners, flavorings, leavening agents, strengthening agents, emulsifying agents, thickening agents, coating agents, gum base agents, antifoaming agents, solvents, and improving agents. These additives may be selectively used depending on the type of food and may be added in appropriate amounts.

In the food composition according to the present invention, the dosage form of the food is not particularly limited as long as it is recognized as a food. The food composition of the present invention may be formulated in various types of food forms and, unlike conventional pharmaceuticals, it is based on food ingredients and thus has the advantage of avoiding side effects that may arise from long-term administration of drugs. Furthermore, due to its excellent portability, the food composition of the present invention may be consumed as a supplement to enhance the effect of preventing or improving PTSD.

(3) feed composition

According to one exemplary embodiment, the present invention provides a feed composition for preventing or improving post-traumatic stress disorder, comprising a PDE5 inhibitor or a pharmaceutically acceptable salt thereof as an active ingredient.

The term "feed", as used herein, may refer to any natural or artificial diet, a meal, or components of the meal, for, or suitable for an animal to eat, ingest, and digest.

In the feed composition according to the present invention, the type of feed is not particularly limited, and any feed commonly used in the art may be employed. Non-limiting examples of the feed include plant-based feeds such as grains, root vegetables, food processing by-products, algae, fibrous materials, pharmaceutical by-products, oils and fats, starches, oil cakes, or grain by-products; and animal-based feeds such as proteins, inorganic materials, oils and fats, minerals, single-cell proteins, zooplankton, or food waste. These may be used alone or in combination of two or more.

2. Method for Preventing or Treating Post-Traumatic Stress Disorder

The present invention provides a method for preventing or treating post-traumatic stress disorder (PTSD), comprising administering to a subject a pharmaceutical composition comprising a phosphodiesterase type 5 (PDE5) inhibitor or a pharmaceutically acceptable salt thereof as an active ingredient.

The term "administration" as used herein refers to introducing the pharmaceutical composition into a subject by an appropriate method.

The term "subject" as used herein refers to any animal including humans that has developed or is susceptible to developing post-traumatic stress disorder, and may specifically include mammals such as humans, mice, rats, and livestock, but is not limited thereto.

The term "pharmaceutically effective amount" as used herein refers to an amount sufficient to treat or prevent a disease at a reasonable benefit-to-risk ratio applicable to medical treatment or prevention. The effective dosage level may be determined based on factors such as the severity of the disease, activity of the drug, age, body weight, health status, and gender of the patient, sensitivity to the drug, timing and route of administration of the composition of the present invention, excretion rate, duration of treatment, drugs used in combination or concurrently with the composition of the present disclosure, and other factors well known in the medical field. The pharmaceutical composition of the present invention may be administered alone or in combination with known therapeutic agents. It is important to administer an amount that achieves maximum efficacy with minimal side effects by considering all of the above factors, which can be readily determined by those skilled in the art.

In the prevention or treatment method according to the present invention, the method may further comprise administering a second active ingredient before, simultaneously with, or after the administration of the pharmaceutical composition comprising a phosphodiesterase type 5 inhibitor or a pharmaceutically acceptable salt thereof as an active ingredient to a subject. The second active ingredient may include an antidepressant, an anxiolytic, or another alpha-1 adrenergic blocker. For example, the antidepressant may include, but is not limited to, a selective serotonin reuptake inhibitor (SSRI), such as sertraline, paroxetine, fluoxetine, citalopram, escitalopram, or fluvoxamine; a serotonin-norepinephrine reuptake inhibitor (SNRI), such as venlafaxine, duloxetine, or desvenlafaxine; a tricyclic antidepressant (TCA), such as amitriptyline or nortriptyline; a monoamine oxidase inhibitor (MAOI), such as phenelzine or tranylcypromine; or an atypical antidepressant, such as mirtazapine, trazodone, or bupropion. The anxiolytic may include, but is not limited to, benzodiazepines such as diazepam, lorazepam, alprazolam, or clonazepam; thienodiazepines such as etizolam or metizolam; or non-benzodiazepine anxiolytics such as buspirone. The alpha-1 adrenergic blocker may include, but is not limited to, prazosin or doxazosin.

In the prevention or treatment method according to the present invention, the administration may include various routes such as oral, intravenous, subcutaneous, intradermal, intranasal, intraperitoneal, intramuscular, or transdermal administration.

In the prevention or treatment method according to the present invention, the dosage of the pharmaceutical composition may be determined by those skilled in the art in consideration of factors such as the purpose of use, severity of the disease, age, body weight, gender, medical history of the patient, or the type of substance used as the active ingredient. For example, the composition of the present invention may be administered at a dose of 0.01 mg/kg to 10 mg/kg, preferably 0.02 mg/kg to 9 mg/kg, and more preferably 0.03 mg/kg to 8 mg/kg based on body weight and although the administration frequency is not particularly limited, it may be administered once daily or in divided doses multiple times a day. The dosage is not intended to limit the scope of the present invention in any respect.

In the prevention or treatment method according to the present invention, the prevention or treatment method may further comprise combination therapy with an additional treatment following administration of the composition. For example, the additional therapy may include, but is not limited to, Eye Movement Desensitization and Reprocessing (EMDR), cognitive behavioral therapy (CBT), exposure therapy, group therapy, or any combination thereof.

## Examples

Hereinafter, the present disclosure will be described in detail through examples. In particular, these examples are solely for describing the present disclosure more specifically, and it is obvious to those skilled in the art that the scope of the present disclosure is not limited by these examples.

### <Experimental Preparation and Methods>

#### 1. Experimental Animals

Twenty-eight male ICR mice, 5 weeks old, weighing approximately 27-30 g, were used. The animals were housed in a controlled environment with a temperature of $26.8 \pm 0.5°C$, relative humidity of $48.4 \pm 1.7\%$, and lighting from 07:00 to 19:00. They were fed a standard diet (AIN-93G diet) and given free access to drinking water. After 7 days of acclimation and habituation, healthy animals were selected for the experiments.

#### 2. Preparation of Test Substances

Mirodenafil was dissolved in 10 mL of distilled water to prepare a 12 mg/kg (MPK) solution. Sildenafil and tadalafil were similarly prepared at 12 MPK each.

#### 3. Group Composition

    1) Control group (n=7): p.o. administration of distilled water (D.W.)
    2) Experimental group 1 (n=7): p.o. administration of mirodenafil (12 MPK)
    3) Experimental group 2 (n=7): p.o. administration of sildenafil (12 MPK)
    4) Experimental group 3 (n=7): p.o. administration of tadalafil (12 MPK)

#### 4. Experimental Procedures

4-1. Drug Administration

Following 7 days of acclimation, the control group received distilled water orally once daily for 15 days, and the experimental groups received their respective drugs orally once daily for 15 days.

4-2. Passive avoidance test

(1) Passive avoidance (Accommodation)

The passive avoidance step-through apparatus was illuminated in the bright chamber at 50% lightness. Mice were placed inside and allowed to move freely between the bright and dark chambers for 5 minutes. After 5 minutes, accommodation was terminated, and mice rested in their home cages.

(2) Passive avoidance (Training)

Mice that completed accommodation were placed in the bright chamber under the same lighting conditions. When the mouse fully entered the dark chamber (all four paws on the floor), an electric shock (0.4 mA, 2 seconds, repeated twice with a 2-second interval) was delivered (maximum latency: 65 seconds, single session). After shock, mice rested in the bright chamber for 10 seconds before training ended. Mice then rested in their home cages.

(3) Passive avoidance (Test)

Mice that completed training were placed again in the bright chamber under the same conditions. The latency to enter the dark chamber (all four paws touching the floor) was recorded without shock (maximum latency: 300 seconds). After the test, mice returned to their home cages.

4-3. Novel object recognition test

Mice were placed in the center of a rat cage and allowed to explore freely for 5 minutes (accommodation). Afterward, mice rested in their home cages.

(1) Novel object recognition (Training1)

Following accommodation, two identical objects (Object A and Object B) of the same color, shape, and size were placed at fixed intervals in the cage. Mice were placed in the cage center and allowed to explore for 5 minutes. After training, mice rested in their home cages.

(2) Novel object recognition (Training2)

Twenty-four hours after Training 1, mice were reintroduced to the cage with the same objects (A and B) spaced similarly. Exploration time of each object was recorded for 5 minutes. Mice then rested.

(3) Novel object recognition (Test)

Twenty-four hours after Training 2, mice were introduced to the cage containing one familiar object (A) and one novel object (C) differing in color, shape, or size. Exploration time was recorded for 5 minutes. The percentage of time spent exploring each object was calculated using the following formula: (Formula to calculate % exploration time for objects)

　　i. Recording Time(ex): Object A: 62s / Object B: 66s
　　ii.

　　Time spent(%)= (Recording time (A) (62) / Recording time (A) + (B) (128)) X 100 = 48%

4-4. Y-maze Test

Mice were placed at the center of a Y-maze and allowed to explore for 8 minutes. Entries were recorded when all four paws fully entered an arm. The maze was cleaned with 70% alcohol between tests. Spontaneous alternation behavior was analyzed using the Yamada statistical method. An alternation was counted when the mouse consecutively entered three different arms. The percentage of spontaneous alternation was calculated as (number of alternations / total arm entries) $\times$ 100.

　　iii. Recording data(ex): BCABCBABCBCABBCACBACCABCABCACBCABC
　　iv.

　　Time spent(%)= (Alternation number of arm entries(22) / Total number of arm entries-2 (31)) X 100 = 66.6%

5. Analysis

Data were expressed as mean $\pm$ standard error of the mean (SEM). Statistical significance was assessed by T-test with thresholds of $P < 0.01$ or $*0.01 < P < 0.05$. Data analysis and plotting were performed using Prism 8 software.

< Results>

1. Passive Avoidance Test

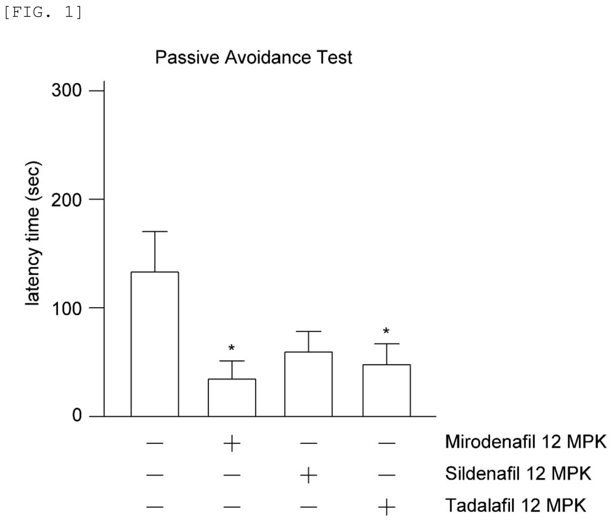

After 15 doses of drug administration followed by electric shock, the latency time was reduced compared to the control: mirodenafil group showed a 73.5% decrease, sildenafil group 55.5%, and tadalafil group 63.2%. This indicates PDE5 inhibitors can block context-specific fear memory (Fig. 1). To demonstrate that this effect was not related to cognitive impairment, NOR and Y-maze results were compared.

2. Novel object recognition test

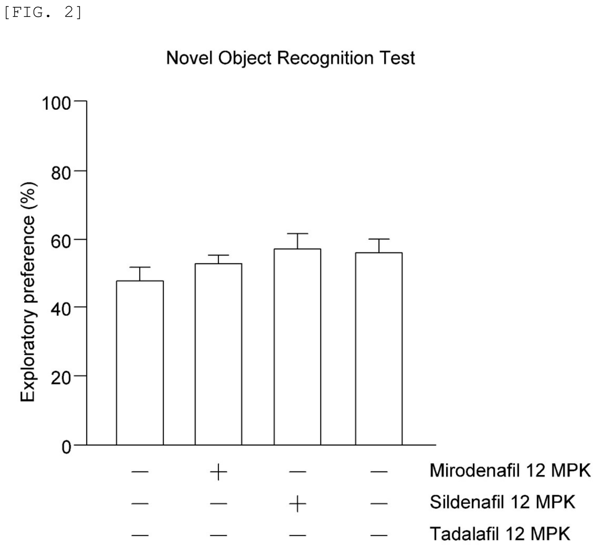

Eight days after drug administration, exploration of novel objects was 49.0% in the control group, while mirodenafil, sildenafil, and tadalafil groups showed increased exploration times of 53.7%, 57.9%, and 57.0%, respectively (Fig. 2). Thus, PDE5 inhibitors blocked fear memory without impairing object recognition and memory in PTSD-induced mice.

3. Y-maze Test

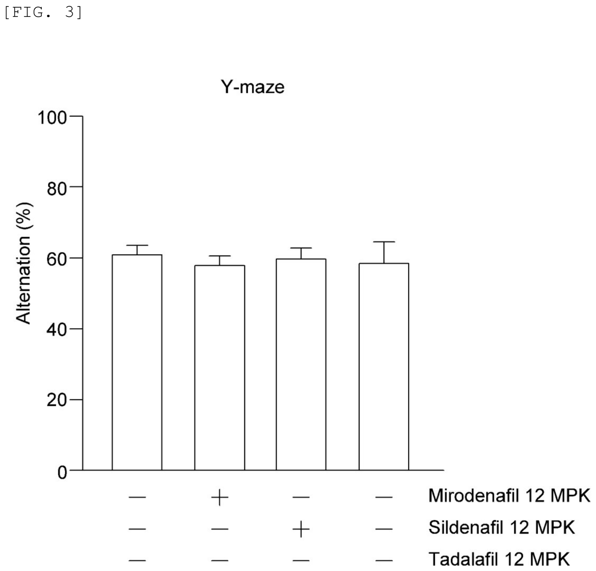

Ten days after drug administration, spontaneous alternation rates were 61.8% for control, 58.4% for mirodenafil, 60.7% for sildenafil, and 59.6% for tadalafil groups, showing similar trends (Fig. 3). This confirms that PDE5 inhibitors block fear memory while preserving spatial recognition and memory in PTSD-induced mice.

As described above, specific parts of the present disclosure have been described in detail; however, it is obvious to those skilled in the art that these specific techniques are merely preferred exemplary embodiments and the scope of the present disclosure is not limited thereto. Accordingly, the actual scope of the present disclosure will be defined by the accompanying claims and equivalents thereof.

**Industrial Applicability**

The phosphodiesterase type 5 inhibitors according to the present invention can selectively block context-specific fear memory while maintaining novel object recognition ability and spatial cognitive function. Therefore, the phosphodiesterase type 5 inhibitors of the present invention exhibit excellent therapeutic effects on post-traumatic stress disorder (PTSD) and are expected to be utilized as active ingredients in pharmaceutical compositions or health functional foods for the development of treatments or ameliorative agents for PTSD.

**Claims**

1.    A pharmaceutical composition for preventing or treating post-traumatic stress disorder, comprising a PDE5 inhibitor or a pharmaceutically acceptable salt thereof as an active ingredient.

2.    The pharmaceutical composition according to claim 1, wherein the PDE5 inhibitor is selected from the group consisting of mirodenafil, sildenafil, vardenafil, tadalafil, udenafil, dasantafil, avanafil, and pharmaceutically acceptable salts thereof.

3.    The pharmaceutical composition according to claim 1, wherein administration of the PDE5 inhibitor comprises oral administration, intravenous injection, subcutaneous injection, intramuscular injection, intraperitoneal injection, endothelial administration, topical administration, intranasal administration, intravaginal administration, intrapulmonary administration, rectal administration, or a combination thereof.

4.    The pharmaceutical composition according to claim 1, wherein a dosage of the PDE5 inhibitor is from 0.01 mg/kg to 10 mg/kg.

5.    The pharmaceutical composition according to claim 1, further comprising a second active agent for preventing or treating post-traumatic stress disorder.

6.    The pharmaceutical composition according to claim 5, wherein the second active agent comprises an antidepressant, an anxiolytic, an alpha-1 blocker, or a combination thereof.

7.    The pharmaceutical composition according to claim 5, wherein the second active agent is selected from the group consisting of sertraline, paroxetine, fluoxetine, citalopram, escitalopram, fluvoxamine, venlafaxine, duloxetine, desvenlafaxine, amitriptyline, nortriptyline, phenelzine, tranylcypromine, mirtazapine, trazodone, bupropion, diazepam, lorazepam, alprazolam, clonazepam, etizolam, metizolam, buspirone, prazosin, doxazosin, and combinations thereof.

8.    The pharmaceutical composition according to claim 1, wherein the pharmaceutical composition is used in conjunction with additional therapies selected from the group consisting of Eye Movement Desensitization & Reprocessing (EMDR), Cognitive Behavioral Therapy (CBT), exposure therapy, group therapy, and combinations thereof.

9. A method for preventing or treating post-traumatic stress disorder, comprising administering to a subject the pharmaceutical composition according to any one of claims 1 to 8.

10. A health functional food for preventing or improving post-traumatic stress disorder, comprising a PDE5 inhibitor or a pharmaceutically acceptable salt thereof as an active ingredient.

11. The health functional food according to claim 10, wherein the PDE5 inhibitor is selected from the group consisting of mirodenafil, sildenafil, vardenafil, tadalafil, udenafil, dasantafil, avanafil, and pharmaceutically acceptable salts thereof.

[FIG. 1]

Passive Avoidance Test

[FIG. 2]

Novel Object Recognition Test

[FIG. 3]

Y-maze

| | | | | |
|---|---|---|---|---|
| — | + | — | — | Mirodenafil 12 MPK |
| — | — | + | — | Sildenafil 12 MPK |
| — | — | — | — | Tadalafil 12 MPK |

**EP 4 656 194 A1**

## INTERNATIONAL SEARCH REPORT

| International application No. |
|---|
| **PCT/KR2024/001065** |

| A. | CLASSIFICATION OF SUBJECT MATTER |
|---|---|

**A61K 31/519**(2006.01)i; **A61K 31/53**(2006.01)i; **A61K 31/4985**(2006.01)i; **A61K 31/522**(2006.01)i; **A61K 31/506**(2006.01)i; **A61P 25/00**(2006.01)i

According to International Patent Classification (IPC) or to both national classification and IPC

| B. | FIELDS SEARCHED |
|---|---|

Minimum documentation searched (classification system followed by classification symbols)

A61K 31/519(2006.01); A61K 31/135(2006.01); A61K 31/428(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 포스포디에스테라제 타입 5 저해제(Phosphodiesterase type 5 inhibitor), 외상후 스트레스 장애(post-traumatic stress disorder)

| C. | DOCUMENTS CONSIDERED TO BE RELEVANT |
|---|---|

| Category* | Citation of document, with indication, where appropriate, of the relevant passages | Relevant to claim No. |
|---|---|---|
| Y | WANG, Chuang et al. Antidepressant-like effects of the phosphodiesterase-4 inhibitor etazolate and phosphodiesterase-5 inhibitor sildenafil via cyclic AMP or cyclic GMP signaling in mice. Metab Brain Dis. 05 April 2014 (Online), vol. 29, pp. 673–682.<br>See abstract; pages 675-676; and figures 1-4. | 1-8,10-11 |
| Y | CHEN, Ling et al. Phosphodiesterase-2 inhibitor reverses post-traumatic stress induced fear memory deficits and behavioral changes via cAMP/cGMP pathway. European Journal of Pharmacology. 01 December 2020 (Online), vol. 891, 173768, pp. 1-9.<br>See abstract; pages 2-3; and figures 1-2 and 5-7. | 1-8,10-11 |
| Y | KR 10-2015-0142012 A (ICAHN SCHOOL OF MEDICINE AT MOUNT SINAI) 21 December 2015 (2015-12-21)<br>See paragraphs [0096], [0129] and [0251]. | 5-8 |
| A | OZBEYLI, Dilek et al. Protective effect of exercise and sildenafil on acute stress and cognitive function. Physiology & Behavior. 28 July 2015 (Online), vol. 151, pp. 230-237.<br>See entire document. | 1-8,10-11 |

☑ Further documents are listed in the continuation of Box C. ☑ See patent family annex.

| * | Special categories of cited documents: |
|---|---|
| "A" | document defining the general state of the art which is not considered to be of particular relevance |
| "D" | document cited by the applicant in the international application |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
|---|---|
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
|---|---|
| **10 May 2024** | **13 May 2024** |

| Name and mailing address of the ISA/KR | Authorized officer |
|---|---|
| **Korean Intellectual Property Office**<br>**Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2022)

**INTERNATIONAL SEARCH REPORT**

| International application No. |
| --- |
| **PCT/KR2024/001065** |

**C.    DOCUMENTS CONSIDERED TO BE RELEVANT**

| Category* | Citation of document, with indication, where appropriate, of the relevant passages | Relevant to claim No. |
| --- | --- | --- |
| A | YU, Yeon Hee et al. Phosphodiesterase-5 Inhibitor Attenuates Anxious Phenotypes and Movement Disorder Induced by Mild Ischemic Stroke in Rats. J Korean Neurosurg Soc. September 2022, vol. 65, no. 5, pp. 665-679.<br>       See entire document. | 1-8,10-11 |

**INTERNATIONAL SEARCH REPORT**

| International application No. |
| --- |
| **PCT/KR2024/001065** |

| Box No. II | Observations where certain claims were found unsearchable (Continuation of item 2 of first sheet) |
| --- | --- |

This international search report has not been established in respect of certain claims under Article 17(2)(a) for the following reasons:

1. ☑ Claims Nos.: **9**
because they relate to subject matter not required to be searched by this Authority, namely:

Claim 9 pertains to a method for treatment of the human body by therapy (PCT Article 17(2)(a)(i) and PCT Rule 39.1(iv)).

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

Form PCT/ISA/210 (continuation of first sheet) (July 2022)

**INTERNATIONAL SEARCH REPORT**
Information on patent family members

International application No.

**PCT/KR2024/001065**

| Patent document cited in search report | | | Publication date (day/month/year) | Patent family member(s) | | | Publication date (day/month/year) |
|---|---|---|---|---|---|---|---|
| KR | 10-2015-0142012 | A | 21 December 2015 | CN | 105377371 | A | 02 March 2016 |
| | | | | CN | 112057441 | A | 11 December 2020 |
| | | | | EP | 2983787 | A1 | 17 February 2016 |
| | | | | EP | 2983787 | A4 | 18 January 2017 |
| | | | | EP | 2983787 | B1 | 02 October 2019 |
| | | | | JP | 2016-516782 | A | 09 June 2016 |
| | | | | JP | 6462663 | B2 | 30 January 2019 |
| | | | | KR | 10-2021-0050599 | A | 07 May 2021 |
| | | | | KR | 10-2022-0044380 | A | 07 April 2022 |
| | | | | KR | 10-2424765 | B1 | 22 July 2022 |
| | | | | US | 10478405 | B2 | 19 November 2019 |
| | | | | US | 11771661 | B2 | 03 October 2023 |
| | | | | US | 2016-0067196 | A1 | 10 March 2016 |
| | | | | US | 2016-0101069 | A1 | 14 April 2016 |
| | | | | US | 2020-0147006 | A1 | 14 May 2020 |
| | | | | US | 2023-0381118 | A1 | 30 November 2023 |
| | | | | WO | 2014-169272 | A1 | 16 October 2014 |

Form PCT/ISA/210 (patent family annex) (July 2022)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- KR 2024001065 W
- KR 1020230009295
- WO 9428902 A
- WO 9519978 A
- KR 0358083
- KR 1020120024807
- KR 1020020031062

**Non-patent literature cited in the description**

- *Journal of Traumatic Stress*, 1991, vol. 4 (3), 419-423
- *Neurosciences (Riyadh)*, 2011, vol. 16 (3), 257-262
- *Nature*, 2002, 674-682
- **BOOLEL, M. et al.** *British Journal of Urology*, 1996, vol. 78, 257-261